# EUROPEAN PATENT APPLICATION

(11) **EP 4 520 322 A1**
(43) Date of publication of application: **12.03.2025**
(21) Application number: 24186360.4
(22) Date of filing: 03.07.2024
(51) Int. Cl.: A61K 9/14, A61K 9/00, A61K 31/4166, A61K 9/16

(54) **PHARMACEUTICAL COMPOSITIONS CONTAINING ENZALUTAMIDE**

(30) Priority: 06.12.2023 IN 202311083018
(71) Applicant: Alfred E. Tiefenbacher (GmbH & Co. KG), 22767 Hamburg (DE)
(72) Inventor: TUNGUTURI, Murali Manohar, 502319 Sangareddy, Telangana (IN); MANKALA, Santhosh Kumar, 502319 Sangareddy, Telangana (IN); JOSHI, Abhay Ramakanth, 502319 Sangareddy, Telangana (IN); NADELLA, Nukendra Prasad, 502319 Sangareddy, Telangana (IN); RACHAKONDA, Veereswara Rao, 502319 Sangareddy, Telangana (IN); HARTWIG, Katharina, 22767 Hamburg (DE); STAVER, Ruslan, 22767 Hamburg (DE)
(74) Representative: Prinz & Partner mbB

(57) **Abstract**

The present invention relates to a pharmaceutical composition comprising or consisting of an amorphous solid dispersion of enzalutamide as active agent in a mixture of hydroxypropyl methylcellulose phthalate and cellulose acetate phthalate as pharmaceutically acceptable excipients, its production process as well as its use in the treatment of a hyperproliferative disorder.

## Description

### Technical Field:

The present invention relates to a pharmaceutical composition comprising or consisting of an amorphous solid dispersion of enzalutamide as active agent in a mixture of hydroxypropyl methylcellulose phthalate and cellulose acetate phthalate as pharmaceutically acceptable excipients, its production process as well as its use in the treatment of a hyperproliferative disorder.

### Background of the invention:

Enzalutamide is an androgen receptor inhibitor indicated for the treatment of patients with castration-resistant prostate cancer and metastatic castration-sensitive prostate cancer. Enzalutamide has the following chemical name: 4-{3-[4-cyano-3-(trifluoromethyl)phenyl]-5,5-dimethyl-4-oxo-2-sulfanylideneimidazolidin-1-yl}-2-fluoro-N-methylbenzamide and structural formula I:

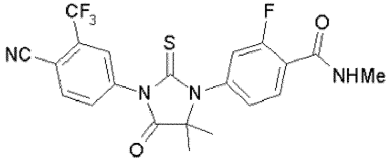

Enzalutamide is presently commercialized under the trade name XTANDI^{®} in dosage forms of 40 mg capsules in many countries worldwide or 40 mg tablets for oral use in EU, US, and Japan (as well as 80 mg tablets in US and Japan), wherein 160 mg enzalutamide dose is administered orally once a day (i.e., once daily four 40 mg Enzalutamide tablets or capsules). Patients receiving enzalutamide should also receive a gonadotropin-releasing hormone (GnRH) analog concurrently. Alternatively, patients receiving enzalutamide should have had bilateral orchiectomy.

As it is known from the European public assessment report (EPAR), enzalutamide is a crystalline solid, which is practically insoluble in aqueous media between pH 1 to 11, but highly permeable and thus, dissolution of the active substance is rate-limiting for bioavailability (BCS class II). Early animal studies demonstrated that bioavailability was greater when enzalutamide was dosed as a solution rather than as a suspension or solid formulation. Therefore, the primary goal of the pharmaceutical development program was to formulate the active substance in the liquid state, encased within a capsule. Caprylocaproyl macroglycerides (CCMG) was identified as the solvent in which the active substance has the greatest solubility, allowing a reasonably sized capsule to be manufactured containing the intended 40 mg strength of enzalutamide. Studies to address the precipitation risk at this concentration were carried out and indicated the long-term solution stability of this formulation. Antioxidants are included to prevent oxidative degradation of the active substance on storage.

According to the prescribing information, the enzalutamide capsules are available as liquid-filled soft gelatin capsules for oral administration, wherein each capsule filling contains 40 mg of enzalutamide as a solution in caprylocaproyl polyoxylglycerides, and two antioxidants: butylated hydroxyanisole (BHA) and butylated hydroxytoluene (BHT). The inactive pharmaceutically acceptable excipients of softgel capsule shell are gelatin, sorbitol sorbitan solution, glycerin, purified water, and titanium dioxide.

Alternatively, the enzalutamide tablets are available as film-coated tablets for oral administration. Each tablet contains 40 mg or 80 mg of enzalutamide. Only 40 mg tablets are marketed in Europe. The inactive pharmaceutically acceptable excipients of the core tablet are hypromellose acetate succinate, microcrystalline cellulose, colloidal silicon dioxide, croscarmellose sodium, and magnesium stearate. The tablet film-coat contains hypromellose, talc, polyethylene glycol, titanium dioxide, and ferric oxide as pharmaceutically acceptable excipients.

XTANDI^{®} tablets contain enzalutamide granules in spray-dried form, wherein the processing liquid in particular contains organic solvents, such as alcohol and/or acetone.

The fact that patients need to take four capsule of large size (20 mm length) or two or four tablets in order to achieve the drug concentration as needed, may lead to a decreased patient compliance, as more than one unit dosage needs to be administered.

Thus, it would be desirable to provide an alternative and/or improved solution for an enzalutamide containing solid pharmaceutical composition, which fulfills the regulatory criteria, such as the criteria on dissolution or stability.

In addition, it would be desirable that the granulation composition of the solid pharmaceutical enzalutamide composition is more economic in costs per unit and/or allows tuning one or more pharmaceutical technological aspects during the production process.

Furthermore, it would be desirable that the granulation composition of the solid pharmaceutical enzalutamide composition is manufactured without using organic solvents, such as alcohol and/or acetone, in order to decrease the negative impact
- on the environment, and/or
- on the health of the production staff, and/or
- on the production settings (risk of explosion, etc.)

Thus, it is an aim of the present invention to provide an alternative and/or improved solid pharmaceutical enzalutamide composition, preferably wherein the enzalutamide containing solid pharmaceutical composition,
- fulfills the regulatory criteria, such as the criteria on dissolution or stability, and optionally
- decreases the negative impact on the environment, and/or on the health of the production staff, and/or on the production settings (risk of explosion, etc.),
- increases patient compliance and is more economic in costs per unit, and/or
- allows tuning one or more pharmaceutical technological aspects during the production process.

### Brief description of the invention:

One or more problems of the present invention is/are solved by the subjects of the present invention as set out in the following description and claims. Advantages (preferred embodiments) are set out in the detailed description hereinafter as well as in the claims.

Accordingly, a first aspect of the present invention relates to a pharmaceutical composition comprising or consisting of an amorphous solid dispersion of enzalutamide as active agent in a mixture of hydroxypropyl methylcellulose phthalate and cellulose acetate phthalate as pharmaceutically acceptable excipients.

A second aspect of the present invention relates to the solid pharmaceutical composition according to the first inventive aspect for use in the treatment of a hyperproliferative disorder, preferably for use in the treatment of
- adult men with non-metastatic castration-resistant prostate cancer (CRPC),
- adult men with metastatic CRPC who are asymptomatic or mildly symptomatic after failure of androgen deprivation therapy in whom chemotherapy is not yet clinically indicated, or
- adult men with metastatic CRPC whose disease has progressed on or after docetaxel therapy.

A third aspect of the present invention relates to a process of production of a pharmaceutical composition, preferably the pharmaceutical composition according to the first inventive aspect, prepared according to hot melt extrusion containing enzalutamide as active ingredient and pharmaceutically acceptable excipients in an amorphous solid dispersion, characterized in that the process comprises or consists of the following steps
a. mixing enzalutamide as active ingredient together with pharmaceutically acceptable excipients comprising or consisting of hydroxypropyl methylcellulose phthalate and cellulose acetate phthalate,
b. heating the mixture of step a) to form a melt and extruding the formed melt followed by subsequent cooling the extrudate to form an amorphous solid dispersion comprising or consisting of enzalutamide, hydroxypropyl methylcellulose phthalate and cellulose acetate phthalate,
c. optionally further processing the amorphous solid dispersion of step b) to form a solid pharmaceutical formulation selected from the group consisting of a powder, granules, pellets, and beads, and
d. optionally further processing the solid pharmaceutical formulation of step c) with one or more further pharmaceutically acceptable excipients selected from the group consisting of diluent agent, flow enhancer agent, disintegrant agent, binder agent, solubilizer agent, and lubricant agent to form a solid pharmaceutical unit dosage formulation selected from the group consisting of tablets, film-coated tablets, capsules, caplets, and multiple unit pellet systems.

A fourth aspect of the present invention relates to a method of treatment or prophylaxis of a hyperproliferative disorder in a patient suffering from, or at risk of, said disorder or condition, which comprises administering to the patient a pharmaceutical composition according to the first or second inventive aspect or prepared according to the third inventive aspect, wherein the patient is selected from an
- adult men with non-metastatic castration-resistant prostate cancer (CRPC),
- adult men with metastatic CRPC who are asymptomatic or mildly symptomatic after failure of androgen deprivation therapy in whom chemotherapy is not yet clinically indicated, or
- adult men with metastatic CRPC whose disease has progressed on or after docetaxel therapy.

The inventive aspects of the present invention as disclosed hereinbefore can comprise any possible (sub-)combination of the inventive aspects and embodiments, in particular any possible (sub-)combination of the embodiments as disclosed in the following detailed description, and/or the claims, provided the resulting combination of features is reasonable to a person skilled in the art.

### Detailed description of the invention:

It has surprisingly been found by the present inventors, that the inventive subject matter provides an alternative and/or improved pharmaceutical enzalutamide composition, wherein the enzalutamide containing pharmaceutical composition of the first or second inventive aspect or prepared according to the third inventive aspect fulfills the regulatory criteria, such as the criteria on dissolution and stability (see Example section, Parts G and H) by mixing enzalutamide as active agent with hydroxypropyl methyl cellulose phthalate and cellulose acetate phthalate respectively as pharmaceutical excipients in order to form the amorphous solid dispersion of the inventive pharmaceutical composition.

The adjustability of the dissolution rate may be increased by adding a pharmaceutically acceptable starch constituent to the combination of hydroxypropyl methylcellulose phthalate and cellulose acetate phthalate in the amorphous solid dispersion.

As the present invention also provides unit dosage formulations having 160 mg enzalutamide in single unit dose, the patient compliance may be increased and / or the production costs may be decreased.

Furthermore, the present invention may at the same time decrease the negative impact on the environment, and/or on the health of the production staff, and/or on the production settings (risk of explosion, etc.) in case the amorphous solid dispersion of the pharmaceutical enzalutamide composition according to the first or second inventive aspect or prepared according to the third inventive aspect is produced without organic solvents. In this regard, the pharmaceutical composition of all aspects and embodiments of the present invention comprising or consisting of the amorphous solid dispersion may be prepared by hot melt extrusion in order to decrease the negative impact on the environment, and/or on the health of the production staff, and/or on the production settings.

In the context of all aspects and embodiments of the present invention, the term / expression *"amorphous solid dispersion"* means that enzalutamide is comprised in the solid dispersion in a non-crystalline state, i.e. enzalutamide in crystalline state may also be comprised. Amorphous enzalutamide dissolves more quickly and/or to a greater extent than crystalline enzalutamide in an aqueous environment, such as an aqueous dissolution medium of an *in vitro* dissolution testing or in the *in vivo* gastrointestinal environment. According to an alternative or additive embodiment of all aspects and embodiments of the present invention, 80 wt.% or more amorphous enzalutamide (i.e., 20 wt.% or less crystalline enzalutamide) based on the weight of the enzalutamide active may be comprised in the amorphous solid dispersion. Alternatively, 90 wt.% or more amorphous enzalutamide (i.e., 10 wt.% or less crystalline enzalutamide) based on the weight of the enzalutamide active ingredient may be comprised in the amorphous solid dispersion. Alternatively, 95 wt.% or more amorphous enzalutamide (i.e., 5 wt.% or less crystalline enzalutamide) based on the weight of the enzalutamide active may be comprised in the amorphous solid dispersion. Alternatively, 99 wt.% or more amorphous enzalutamide (i.e., 1 wt.% or less crystalline enzalutamide) based on the weight of the enzalutamide active may be comprised in the amorphous solid dispersion. The aforementioned weight ranges are to be regarded to include a measured value ± 5% tolerance to fall within range of specified weight ranges.

According to an alternative or additive embodiment of all aspects and embodiments of the present invention, the amorphous solid dispersion of the pharmaceutical composition may further comprise starch as a pharmaceutically acceptable excipient. As set out hereinbefore, such a constituent may optimize the dissolution or stability properties of the pharmaceutical composition of all aspects and embodiments of the present invention, in particular optimize the adjustability of dissolution rate. This effect is presently believed to be due to the corresponding increase of the free volume between the polymer chains to greater chain mobility and/or due to the function of starch as disintegrant and/or due to the function of starch as a foam-forming agent, resulting in increased specific surface area and improved milling efficiency due to porous nature and morphology changes, and/or due to the function of starch as pore-forming agent to increase the intake of aqueous solution into in order to increase dissolution of enzalutamide as active agent.

In this regard, the starch constituent contained in the amorphous solid dispersion of the pharmaceutical composition of all aspects and embodiments of the present invention may be selected from one or more pharmaceutically acceptable excipients including the group consisting of maize starch, wheat starch, and potato starch. In particular, maize starch may be selected as the starch constituent of all aspects and embodiments of the present invention.

According to an alternative or additive embodiment of all aspects and embodiments of the present invention, the amorphous solid dispersion may further comprise a plasticizer as a pharmaceutically acceptable excipient. Such a constituent may optimize the dissolution or stability properties of the pharmaceutical composition of all aspects and embodiments of the present invention, in particular optimize the adjustability of dissolution rate. This effect is presently believed to be due to the corresponding increase of the free volume between the polymer chains to greater chain mobility and therefore to increase the intake of aqueous solution in order to increase dissolution of enzalutamide as active agent.

In this regard, the plasticizer constituent contained in the amorphous solid dispersion of the pharmaceutical composition of all aspects and embodiments of the present invention may be selected from one or more pharmaceutically acceptable excipients including the group consisting of triethyl citrate, triacetin, dibutyl sebacate, diethyl phthalate, glyceryl monostearate, glycerin, polyethylene glycol, and propylene glycol. In particular, triethyl citrate may be selected as the plasticizer constituent of all aspects and embodiments of the present invention.

According to an alternative or additive embodiment of all aspects and embodiments of the present invention, the amorphous solid dispersion may further comprise an antioxidant as a pharmaceutically acceptable excipient. Such a constituent may further optimize the dissolution or stability properties of the pharmaceutical composition of all aspects and embodiments of the present invention, in particular the chemical stability properties of enzalutamide.

In this regard, the antioxidant constituent contained in the amorphous solid dispersion of the pharmaceutical composition of all aspects and embodiments of the present invention may be selected from one or more pharmaceutically acceptable excipients including propyl gallate, butylated hydroxyanisole (BHA), butylated hydroxytoluene (BHT), ascorbic acid, and ascorbyl palmitate. In particular, propyl gallate is selected as antioxidant with respect to all aspects and embodiments of the present invention.

In general, the amorphous solid dispersion of the pharmaceutical composition of all aspects and embodiments of the present invention may contain any suitable amount of enzalutamide as active agent, and the pharmaceutical excipients hydroxypropyl methylcellulose phthalate, cellulose acetate phthalate and optionally starch excipient and/or optionally plasticizer excipient and/or optionally antioxidant excipient to form the pharmaceutical composition according to all aspects and embodiments of the present invention. According to an alternative or additive embodiment of all aspects and embodiments of the present invention, the amorphous solid dispersion may contain:
a. an amount of enzalutamide as active agent in the range of 10 to 25 wt.%, preferably 15 to 20 wt.%, more preferably 15 to 16 wt.% respectively based on the total weight of the amorphous solid dispersion, and/or
b. an amount of hydroxypropyl methylcellulose phthalate in the range of 30 to 70 wt.%, preferably 55 to 70 wt.%, more preferably 56 to 64 wt.% or 59 to 61 wt.%, in particular 56 wt.%, 57 wt.%, 58 wt.%, 59 wt.%, 60 wt.%, 61 wt.%, 62 wt.%, 63 wt.%, 64 wt.% or 65 wt.% alternatively 40 to 60 wt.%, alternatively 45 to 55 wt.% respectively based on the total weight of the amorphous solid dispersion, and/or
c. an amount of cellulose acetate phthalate in the range of 10 to 50 wt.%, preferably 10 to 20 wt.%, more preferably 11 to 19 wt.% or 14 to 16 wt.%, in particular 12 wt.%, 13 wt.%, 14 wt.%, 15 wt.%, 16 wt.%, 17 wt.%, 18 wt.%, or 19 wt.%, alternatively 20 to 35 wt.%, or alternatively 25 to 30 wt.% respectively based on the total weight of the amorphous solid dispersion, and/or
d. a ratio between hydroxypropyl methylcellulose phthalate and cellulose acetate phthalate in the range of 6 : 1 to 0.5 : 1, preferably 4.5 : 1 to 3.5 : 1, alternatively 1.5: 1 to 2.5 : 1 respectively based on the total weight of the amorphous solid dispersion, and/or
e. an amount of starch in the range of 0 to 10 wt.%, preferably 3 to 8 wt.%, more preferably 3 to 4 wt.%, alternatively 4 to 6 wt.% respectively based on the total weight of the amorphous solid dispersion, and/or
f. an amount of plasticizer in the range of 0 to 10 wt.%, preferably 3 to 8 wt.%, more preferably 4 to 6 wt.% respectively based on the total weight of the amorphous solid dispersion, and/or
g. a ratio between starch to plasticizer in the range of 8 : 0 to 0 : 10, preferably 0.5 : 1 to 1.5 : 1, more preferably 1 : 1.5 to 1 : 1, in particular 1 : 1.1, 1 : 1.2, 1 : 1.3, or 1 : 1.4 respectively based on the total weight of the amorphous solid dispersion, and/or
h. an amount of antioxidant, such as propyl gallate, in a range of 0 to 1 wt.%, preferably 0.1 to 0.8 wt.%, more preferably 0.2 to 0.7 wt.% respectively based on the total weight of the amorphous solid dispersion.

The ratio (in weight) between enzalutamide as active agent and the mixture of the polymeric constituents of the amorphous solid dispersion, namely hydroxypropyl methylcellulose phthalate and cellulose acetate phthalate, is surprisingly increased in comparison to other formulations generally used for hot melt extrusion. The present ratio range of enzalutamide to the mixture of hydroxypropyl methylcellulose phthalate and cellulose acetate phthalate in the amorphous solid dispersion is in the range of 1 : 4 to 1 : 6, in particular being 1 : 5 respectively based on the total weight of the amorphous solid dispersion.

This surprising increase in ratio between active agent and the mixture of hydroxypropyl methylcellulose phthalate and cellulose acetate phthalate as polymeric constituents of the amorphous solid dispersion as well as the increase in ratio between hydroxypropyl methylcellulose phthalate and cellulose acetate phthalate is presently believed to further improve the dissolution properties of the inventive pharmaceutical composition of all aspects and embodiments of the present invention.

The aforementioned weight ranges of amounts and ratios are to be regarded to include a measured value ± 5% tolerance to fall within range of specified weight ranges.

The respectively given amounts and ratios facilitate to optimize the (improved) regulatory criteria, such as with respect to dissolution and stability, in particular for a unit dose having more than 80 mg enzalutamide per unit, in particular having 120 mg enzalutamide per unit dosage or 160 mg enzalutamide per unit dosage.

In general, the amorphous solid dispersion of the pharmaceutical composition of all aspects and embodiments of the present invention may be prepared by any suitable pharmaceutical production process, in particular including spray drying, drum drying or co-precipitation. As set out hereinbefore, such a production process is even more preferred, in case less or no organic solvents are used in the production process. Instead, an aqueous solvent can be chosen including purified water (e.g., Water, Purified, *Ph. Eur*.)*.*

In this regard, the amorphous solid dispersion of the pharmaceutical composition of all aspects and embodiments of the present invention may be prepared by spray granulation on a pharmaceutically acceptable carrier or wet granulation with a pharmaceutically acceptable carrier in particular when using no organic solvent. According to an alternative or additive embodiment thereof, the pharmaceutically acceptable carrier may be selected from one or more pharmaceutically acceptable excipients selected from the group of diluents, disintegrants, and silica-based adsorbents. According to an alternative or additive embodiment thereof, the silica-based adsorbent may be selected from one or more pharmaceutically acceptable excipients of the group consisting of a mesoporous silica, colloidal anhydrous silica, magnesium aluminosilicate, and calcium silicate.

As set out herein before, the pharmaceutical composition according to all inventive aspects and embodiments can consist of the amorphous solid dispersion, i.e., the amorphous solid dispersion represents the pharmaceutical composition of all aspects and embodiments of the present invention. Alternatively, the amorphous solid dispersion may be comprised in the pharmaceutical composition of all aspects and embodiments of the present inventions, i.e., in addition to the amorphous solid dispersion also further pharmaceutical excipients are comprised in the pharmaceutical composition of all aspects and embodiments of the present invention. In this regard, the amorphous solid dispersion of the pharmaceutical composition of all aspects and embodiments of the present invention may be further processed to form a solid pharmaceutical formulation selected from the group consisting of powder, granules, pellets, and beads. Such a solid pharmaceutical formulation may generally contain more than one unit dose and may be partitioned into the respective unit dosages.

According to an alternative or additive embodiment thereof, the amorphous solid dispersion or the solid pharmaceutical formulation of the pharmaceutical composition of all aspects and embodiments of the present invention may still be further processed with one or more further pharmaceutically acceptable excipients selected from the group consisting of diluent agent, flow enhancer agent, disintegrant agent, binder agent, solubilizer agent, and lubricant agent, to form a solid pharmaceutical unit dosage formulation selected from the group consisting of tablets, film-coated tablets, capsules, caplets, and multiple unit pellet systems.

In general, the unit dosage of enzalutamide as active agent in the pharmaceutical composition of all aspects and embodiments of the present invention may cover any suitable dosage to treat the hyperproliferative disorder or to administer a prophylactic dosage to the respective patient. According to an alternative or additive embodiment of all aspects and embodiments of the present invention, the unit dosage form of the pharmaceutical composition may contain 40 mg, 80 mg, 120 mg or 160 mg enzalutamide as active ingredient per unit dosage form. In particular, the unit dosage form of all aspects of the present invention comprises 160 mg enzalutamide as active ingredient per unit dosage form.

According to an alternative or additive embodiment of all aspects and embodiments of the present invention, the solid pharmaceutical formulation or the solid pharmaceutical unit dosage form of the pharmaceutical composition may comprise one or more further pharmaceutically acceptable excipients selected from the group consisting of (silicified) microcrystalline cellulose, hydroxypropyl cellulose, low-substituted hydroxypropyl cellulose, methylcellulose, hydroxypropyl methylcellulose, hydroxypropyl methylcellulose acetate succinate, croscarmellose sodium, maltodextrin, starch, sodium starch glycolate, pregelatinized starch, crospovidone, lactose monohydrate, mannitol, magnesium stearate, colloidal anhydrous silica, sodium stearyl fumarate, and sodium carbonate, more preferably (silicified) microcrystalline cellulose, low-substituted hydroxypropyl cellulose, lactose monohydrate, magnesium stearate, colloidal anhydrous silica, croscarmellose sodium, and crospovidone.

According to an alternative or cumulative embodiment, the solid pharmaceutical formulation or the solid pharmaceutical unit dosage form of the pharmaceutical composition of all aspects and embodiments of the present invention, in particular a tablet composition, comprises or consists of enzalutamide, hydroxypropyl methylcellulose phthalate, cellulose acetate phthalate, maize starch, triethyl citrate respectively forming the amorphous solid dispersion and silicified microcrystalline cellulose, low-substituted hydroxypropyl cellulose, magnesium stearate, crospovidone and lactose monohydrate. In this embodiment,
a) the amount of enzalutamide as active agent is in the range of 15 to 16 wt.% respectively based on the total weight of the amorphous solid dispersion, and/or is 160 mg per unit dosage form of the solid pharmaceutical formulation and/or
b) the amount of hydroxypropyl methylcellulose phthalate is in the range of 59 to 61 wt.%, in particular 60 wt.% or 61 wt.% respectively based on the total weight of the amorphous solid dispersion, and/or
c) the amount of cellulose acetate phthalate is in the range of 14 to 16 wt.%, in particular 14 wt.%, 15 wt.%, or 16 wt.% respectively based on the total weight of the amorphous solid dispersion, and/or
d) the ratio between hydroxypropyl methylcellulose phthalate and cellulose acetate phthalate is in the range of 4.5 : 1 to 3.5 : 1 respectively based on the total weight of the amorphous solid dispersion, and/or
e) the amount of maize starch is in the range of 3 to 4 wt.% respectively based on the total weight of the amorphous solid dispersion, and/or
f) the amount of triethyl citrate as plasticizer is in the range of 4 to 6 wt.% respectively based on the total weight of the amorphous solid dispersion, and/or
g) the ratio between maize starch to triethyl citrate as plasticizer in the range of 1 : 1.5 to 1 : 1.2 respectively based on the total weight of the amorphous solid dispersion, and/or
h) no antioxidant is used in the amorphous solid dispersion, and/or
i) the ratio between enzalutamide as active agent and the mixture of hydroxypropyl methylcellulose phthalate and cellulose acetate phthalate as polymeric constituents of the amorphous solid dispersion is in the range of 1 : 4 to 1 : 6, preferably 1 : 5 respectively based on the total weight of the amorphous solid dispersion, and/or
j) the amount of silicified microcrystalline cellulose is in the range of 8 to 12 wt.%, in particular 10 wt.% respectively based on the total weight of the solid pharmaceutical composition and/or
k) the amount of low-substituted hydroxypropyl cellulose is in the range of 1 to 5 wt.%, preferably 3 to 4 wt.% respectively based on the total weight of the solid pharmaceutical composition and/or
l) the ratio between silicified microcrystalline cellulose and low-substituted hydroxypropyl cellulose is in the range of 3.5 : 1 to 2 : 1, preferably 3.1 : 1 to 2.5 : 1, in particular 2.5 : 1, 2.6 : 1, 2.7 : 1, 2.8 : 1, 2.9 : 1, 3.0 : 1, or 3.1 : 1 respectively based on the total weight of the solid pharmaceutical composition and/or
m) the amount of magnesium stearate is in the range of 0.1 to 1 wt.%, preferably in the range of 0.5 to 0.8 wt.%, in particular 0.6 or 0.7 wt.% respectively based on the total weight of the solid pharmaceutical composition and/or
n) the amount of crospovidone is in the range of 1 to 10 wt.%, preferably in the range of 5 to 8 wt.%, in particular 6 wt.% or 7 wt.% respectively based on the total weight of the solid pharmaceutical composition and/or
o) the amount of lactose monohydrate is in the range of 1 to 10 wt.%, preferably in the range of 5 to 8 wt.%, in particular 6 wt.% or 7 wt.% respectively based on the total weight of the solid pharmaceutical composition.

As set out hereinbefore, in particular the ratio by weight between enzalutamide as active agent to the mixture of hydroxypropyl methylcellulose phthalate and cellulose acetate phthalate as polymeric constituents of the amorphous solid dispersion in the range of 1 : 4 to 1 : 6, preferably 1 : 5 and the ratio by weight between hydroxypropyl methylcellulose phthalate and cellulose acetate phthalate as such in the range of 4.5 : 1 to 3.5 : 1 respectively based on the total weight of the amorphous solid dispersion is presently believed to further improve the dissolution properties of the inventive pharmaceutical composition of all aspects and embodiments of the present invention. This advantage in particucular cumulatively or alternatively applies, in case the inventive pharmaceutical composition is a tablet composition, preferably a film coated tablet composition and includes 160 mg enzalutamide as active agent per unit dosage form. Furthermore, this advantage cumulatively or alternatively applies in case the amount of enzalutamide as active agent is in the range of 15 to 16 wt.% respectively based on the total weight of the amorphous solid dispersion. Furthermore, this advantage cumulatively or alternatively applies in case the amount of hydroxypropyl methylcellulose phthalate is in the range of 59 to 61 wt.%, in particular 60 wt.% or 61 wt.% respectively based on the total weight of the amorphous solid dispersion. Furthermore, this advantage cumulatively or alternatively applies in case the amount of cellulose acetate phthalate is in the range of 14 to 16 wt.%, in particular 14 wt.%, 15 wt.%, or 16 wt.% respectively based on the total weight of the amorphous solid dispersion.

According to an alternative or additive embodiment of all aspects and embodiments the present invention, the pharmaceutical composition may further comprise or consist of a therapeutically effective amount of one or more additional active ingredients. The one or more additional active ingredients may be comprised in the granular composition or - in case of prevention of interaction between the one or more further active ingredients and enzalutamide - in the extragranular composition.

According to an alternative or additive embodiment of all aspects and embodiments of the present invention, the inventive pharmaceutical composition may be con-currently or sequentially co-administered with a separate pharmaceutical composition containing the one or more additional active ingredients. Co-administering the inventive pharmaceutical composition and the separate pharmaceutical composition containing the additional active ingredient(s) may be preferred in cases, where, e.g., the different active ingredients may interact and in particular, where the dissolution and/or stability criteria may be at risk to be negatively affected. In addition, co-administration may be preferred in cases, where, e.g., enzalutamide of the inventive pharmaceutical composition and the additional active ingredient(s) of the separate formulation may require different administration conditions, e.g., where the additional separate active ingredients may be administered not once a day, but two or more times a day, or where the dosage of the additional separate active ingredient(s) may need to be individually adapted in view of physiological constitution of the patient, or where the additional separate active ingredient may not be administered orally, but, e.g., nasal, transdermal, intramuscular (*i.m*.) or intravenous (*i.v.*), or where the additional separate active ingredient may not be administered in form of a solid composition, in particular a tablet composition, but, e.g., in liquid form.

In this regard, the one or more additional active ingredients contained in the pharmaceutical composition of all aspects and embodiments of the present invention or contained in a separate pharmaceutical formulation may preferably be selected from constituents, which are effective in the treatment or prophylaxis of a hyperproliferative disorder, preferably wherein the patient is an
- adult men with non-metastatic castration-resistant prostate cancer (CRPC),
- adult men with metastatic CRPC who are asymptomatic or mildly symptomatic after failure of androgen deprivation therapy in whom chemotherapy is not yet clinically indicated, or
- adult men with metastatic CRPC whose disease has progressed on or after docetaxel therapy.

Accordingly, the one or more additional active ingredients may be selected from the group of Gonadotropin Releasing Hormone (GnRH) antagonists, such as degarelix; Luteinizing Hormone Releasing Hormone (LHRH) agonists, Gonadotropin Releasing Hormone (GnRH) agonists, such as leuproreline busereline, gosereline, and triptoreline; non-steroidal antiandrogens, such as bicalutamide, flutamide and cyproteronacetate; and cytostatic agents, such as estramustine and docetaxel.

The properties or the physical and chemical stability of the pharmaceutical composition according to all aspects and embodiments of the present invention may be tested in conventional manner, e.g. by measurement of appearance, hardness (or resistance to crushing), disintegration time, dissolution, friability, water content, assay for enzalutamide and/or its degradation products (related substances), and/or uniformity of dosage units or mass after storage at controlled storage conditions; e.g. at long-term and/or accelerated conditions according to the guidelines of the International Council for Harmonisation of Technical Requirements for Pharmaceuticals for Human Use (ICH) Q1A(R2) (i.e. at 25 °C / 60 % relative humidity (RH) and/or at 40 °C / 75 % RH). These tests shall be performed according to applicable pharmaceutical regulatory standards as described, e.g., in ICH guidelines or European Medicines Agency (EMA) guidelines or the European Pharmacopeia (Ph. Eur.) and/or US Pharmacopeia (USP).

At least some of these attributes, i.e., properties or physical and chemical stability, preferably most of these attributes and most preferably all of these attributes of the pharmaceutical composition of all aspects and embodiments of the present invention, in particular inventive tablet formulation is stable over time and different controlled storage conditions. In an alternative or additive embodiment of all aspects and embodiments of the present invention, the dissolution (profile) of the inventive pharmaceutical tablet composition according to the present invention, e.g. a tablet or film-coated tablet, is stable over 1, 2, 3, 4, 5, 6 or more, preferably at least 6 months when stored preferably in PVC/PCTFE-Alu blisters or HOPE bottles at long-term or accelerated storage conditions, i.e. 25 °C / 60 % RH or 40 °C / 75 % RH. In particular, dissolution and further additional attributes such as, e.g., assay, related substances or uniformity of dosage units or mass may also be stable after storage over 1, 2, 3, 4, 5, 6 or more, preferably at least 6 months when stored at long-term or accelerated storage conditions. The term "stable" in the context of the present invention means that a measured value ± 5% tolerance falls within range of specified values determined in accordance with a respective applicable regulatory guideline, e.g., the European Pharmacopeia or USP.

In general, the pharmaceutical composition according to all aspects and embodiments of the present invention can have any suitable constitution and/or shape. According to an alternative embodiment of the pharmaceutical composition of all aspects and embodiments of the present invention, in particular the tablet formulation and further in particular the film-coated tablet, may vary in shape and may be, e.g., round, oval, oblong, cylindrical, caplet shaped or any other suitable shape, preferably it is round or oval shaped. Furthermore, the pharmaceutical composition according to all aspects and embodiments of the present invention may comprise means for dividing the tablet, such as scores or engravings.

In general, the pharmaceutical composition according to all aspects and embodiments of the present invention can have any suitable dimensions. According to an alternative embodiment of the pharmaceutical composition of all aspects and embodiments of the present invention, in particular the tablet formulation and further in particular the film-coated tablet, may have a diameter ranging between 3.5 and 21.5 mm and most preferably between 7.5 and 12 mm respectively for round tablets, and a dimension between 9 x 3 mm and 17 x 9 mm, alternatively up to 21.5 x 10.5 mm respectively for oval tablets. According to an alternative embodiment of all aspects and embodiments of the present invention as described herein, the thickness of the pharmaceutical composition, in particular the tablet formulation and further in particular the film-coated tablet, may range from 3 to 10 mm, preferably between 4 and 9 mm.

According to an alternative example embodiment of the pharmaceutical composition of all aspects and embodiments of the present invention, in particular the tablet formulation and further in particular the film-coated tablet, may have the following dimensions:
- Enzalutamide 40 mg per tablet: round tablet, 10 mm in diameter,
- Enzalutamide 80 mg per tablet: oval tablet, 17 mm long and 9 mm in width,
- Enzalutamide 120 mg per tablet: oval tablet, 19 mm long and 9.5 mm in width, and
- Enzalutamide 160 mg per tablet: oval tablet, 21.5 mm long and 10.5 mm in width.

The given dimensions of the pharmaceutical composition according to all aspects and embodiments of the present invention, in particular of the tablet formulation, more in particular the film-coated tablet formulation are not regarded to be strict, but to include a production tolerance of respectively ± 5% to fall within the specified values.

The pharmaceutical composition according to all aspects and embodiments of the present invention, in particular the tablet composition, more in particular the film-coated tablet, may generally exhibit any suitable hardness (or resistance to crushing). According to an alternative example embodiment of all aspects and embodiments of the present invention as described herein, the pharmaceutical composition, in particular the tablet formulation and further in particular the film-coated tablet, may have a hardness (or resistance to crushing) of at least 40 N, alternatively from 80 N to 250 N, preferably 100 N to 200 N. In general, the higher the hardness score, the less prone to crushing is the pharmaceutical composition according to all aspects and embodiments of the present invention. In view of interaction with the regulatory requirement of dissolution and disintegration time, the upper level of the hardness score is limited and needs to be adapted in accordance with the given subranges.

Furthermore, the pharmaceutical composition according to all aspects and embodiments of the present invention, in particular the tablet composition, preferably the film-coated tablet, may have a suitable disintegration time. According to an alternative example embodiment of all aspects and embodiments of the present invention as described herein, the pharmaceutical composition, in particular the tablet formulation and further in particular the film-coated tablet, may have a disintegration time of up to 10 minutes, alternatively of up to 5 minutes, in particular between 1 and 4 minutes. The less disintegration time, the quicker may the active agent be dissolved in the aqueous environment and, thus, may resorbed.

According to an alternative or additive embodiment, the pharmaceutical composition according to all aspects and embodiments of the present invention, in particular the tablet composition, preferably the film-coated tablet, may have a suitable dissolution in Quality Control (QC) media of not less than 75 wt.% of the labelled amount of enzalutamide dissolved within 30 minutes.

According to an alternative or additive embodiment, the pharmaceutical composition according to all aspects and embodiments of the present invention, in particular the tablet composition, preferably the film-coated tablet, may be colored and/or marked so as to impart an individual appearance and to make them instantly recognizable. The use of dyes can generally serve to enhance the appearance as well as to identify the inventive pharmaceutical composition. Dyes suitable for use in pharmacy typically include carotenoids, iron oxides or chlorophyll. The pharmaceutical composition according to all aspects and embodiments of the present invention, in particular the tablet composition, preferably the film-coated tablet may be marked using an imprint code.

The pharmaceutical composition according to all aspects and embodiments of the present invention, in particular the tablet composition, preferably the film-coated tablet may be packed in any conventional packaging known in the art. According to an alternative or additive embodiment, the pharmaceutical composition according to all aspects and embodiments of the present invention, in particular the tablet composition, preferably the film-coated tablet, may be packed for example in blisters, polypropylene or polyethylene (e.g., HDPE, high density polyethylene) containers or glass bottles. According to an alternative or additive embodiment, the pharmaceutical composition according to all aspects and embodiments of the present invention, in particular the tablet or capsule composition, may be packed in a blister known in the art, e.g. sealed aluminum blister (Alu-Alu), Aluminum / Aluminum peel-push blisters or PVC/PE/PVDC/aluminum blisters or PVC/PCTFE/aluminum blisters or HOPE bottles having a shelf life of up to 3 years.

The present invention is explained further with the aid of the following nonlimiting examples, illustrating the parameters of and compositions employed within the present invention. Unless stated otherwise, all data, in particular percentages, parts and ratios are by weight.

According to the present invention, the individual features of the exemplary embodiments of the inventive pharmaceutical tablet composition and the inventive production process as disclosed in the detailed description or claims of the present application can respectively be separately combined with single features or (sub)combination of features of the further exemplary embodiments herein below. In other words, a person skilled in the art is directly taught to combine each of the pharmaceutical constituents, their amount (weight) and/or their weight ratios separately or in each of the combinations with the pharmaceutical composition according to all aspects and embodiments of the present invention as disclosed in the description hereinbefore and/or claims. Furthermore, a person skilled in the art is directly taught to combine each of the production process conditions, in particular the production process conditions of preparing the amorphous solid dispersion by either hot melt extrusion or by spray granulation / wet granulation separately or in each of the combinations with the production process according to all aspects and embodiments of the present invention as disclosed in the description hereinbefore and/or claims.

### Examples:

### Part A: General procedure for the production of an amorphous solid dispersion containing enzalutamide by hot melt extrusion and optional subsequent further processing

### Stage A: (Dry Mix)

1. Dry mixing of enzalutamide, polymeric constituents hydroxypropyl methylcellulose phthalate and cellulose acetate phthalate as well as optionally further pharmaceutical excipients, such as starch (e.g., maize starch), plasticizer (e.g., triethyl citrate or triacetin) and antioxidant (e.g., propyl gallate).

### Stage B: (Hot Melt Extrusion)

2. Mixed materials of step-1 are loaded into a hot melt extruder (e.g., model: 11 HME; manufacturer: ThermoFisher) and optionally further mixing for a suitable amount of time to facilitate homogeneous dispersion (dependent on the used apparatus up to 10 minutes, preferably less), applying a suitable heating temperature to facilitate melting of the polymeric constituents of step-1 without degrading the active agent enzalutamide, preferably the applied temperature is in the range of 120°C to 170°C for a suitable amount of time, such as up to 5 minutes, and subsequent kneading of the molten mass to form the extrudate comprising the amorphous solid dispersion containing enzalutamide.

3. Optionally milling the extrudate of step-2 in a suitable apparatus Hammer mill (e.g., model: HMI; manufacturer: Frewitt) using 0.3 mm round screen to form a solid pharmaceutical formulation, such as selected from the group consisting of powder, granules, pellets, and beads.

### Optional Stage C: (Blending and further processing)

4. Blending and lubricating extragranular materials of pharmaceutically acceptable excipients, such as selected from the group consisting of diluent agent, flow enhancer agent, disintegrant agent, binder agent, solubilizer agent, and lubricant agent with the amorphous solid dispersion extrudate of step-2 or the solid pharmaceutical formulation of step-3 and further processing the blend to form a solid pharmaceutical unit dosage formulation, such as selected from tablets, film-coated tablets, capsules, caplets, multiple unit pellet systems.

5. As an example, the blend of step-4 is compressed into cores of tablets, e.g., on a rotary tablet compression machine using suitable tooling and parameters.

### Optional Stage D: Film-Coating

6. Film coating the core tablets of step-5, e.g., using a suitable coating pan, with a suitable film coating constituent, e.g., a suitable Opadry^{®} suspension.

### Part B: Process of production of comparative pharmaceutical tablet formulations containing enzalutamide by hot melt extrusion

The following comparative pharmaceutical tablet formulations C1-C4 were produced in accordance with the general procedure as set out in Part A above.

### Example C1: Comparative solid pharmaceutical unit dosage formulation, namely tablet formulation containing 160 mg enzalutamide per tablet.

| **Ingredients** | **C1 (mg/unit)** |
|---|---|
| **Stage-A: (Dry Mix)** | |
| Enzalutamide, *In-house* | 160.224 |
| Hydroxypropyl Methylcellulose Acetate Succinate (HPMCAS MMP), *Ph. Eur.* | 480 |
| Colloidal anhydrous silica (Aerosil^{®} 200), *Ph. Eur.* | 5 |
| Triethyl citrate, *Ph. Eur.* | 25 |

| **Stage-B: (Hot Melt Extrusion)** | |
|---|---|
| **Total weight of Extrudes** | **670.224** |

| **Stage-C (Blending and Tablet Compression)** | |
|---|---|
| Silicified microcrystalline cellulose, *Ph. Eur.* | 665.776 |
| Magnesium stearate, *Ph. Eur.* | 14 |
| Croscarmellose sodium, *Ph. Eur.* | 50 |
| **Total weight of tablet (mg)** | **1,400.00** |

In view that the hardness of the manufactured core tablets was poor, the coating step was not performed.
Thickness: 7.7 mm
Hardness: 65-81 N

### Example C2: Comparative solid pharmaceutical unit dosage formulation, namely tablet formulation containing 160 mg enzalutamide per tablet.

| **Ingredients** | **C2 (mg/unit)** |
|---|---|
| **Stage-A: (Dry Mix)** | |
| Enzalutamide, *In-house* | 160.224 |
| Hydroxypropyl Methylcellulose Phthalate (HPMCP HP-55), *Ph. Eur.* | 480 |
| Colloidal anhydrous silica (Aerosil^{®} 200), *Ph. Eur.* | 5 |
| Triethyl citrate, *Ph. Eur.* | 56 |

| **Stage-B: (Hot Melt Extrusion)** | |
|---|---|
| **Total weight of Extrudes** | **701.224** |

| **Stage-C (Blending and Tablet Compression)** | |
|---|---|
| Silicified microcrystalline cellulose, *Ph. Eur.* | 638.776 |
| Magnesium stearate, *Ph. Eur.* | 10 |
| Croscarmellose sodium, *Ph. Eur.* | 50 |
| **Total weight of tablet (mg)** | **1,400.00** |

In view that the hardness of the manufactured core tablets was poor, the coating step was not performed.
Thickness: 7.7 mm
Hardness: 72.6 N

### Example C3: Comparative solid pharmaceutical unit dosage formulation, namely tablet formulation containing 160 mg enzalutamide per tablet.

| **Ingredients** | **C3 (mg/unit)** |
|---|---|
| **Stage-A: (Dry Mix)** | |
| Enzalutamide, *In-house* | 160.224 |
| Cellulose Acetate Phthalate (CAP), *Ph. Eur.* | 640 |

| **Stage-B: (Hot Melt Extrusion)** | |
|---|---|
| **Total weight of Extrudes** | **800.224** |

| **Stage-C (Blending and Tablet Compression)** | |
|---|---|
| Colloidal anhydrous silica (Aerosil^{®} 200), *Ph. Eur.* | 6 |
| Croscarmellose sodium, *Ph. Eur.* | 73.776 |
| Microcrystalline cellulose, *Ph. Eur.* | 73 |
| Sodium stearyl fumarate, *Ph. Eur.* | 6 |
| **Total weight of tablet (mg)** | **959** |

| **Stage-D (Film Coating)** | |
|---|---|
| Opadry^{®} white, *In-house* | 30.000 |
| Water, Purified, *Ph. Eur.* | q.s. |
| **Film-coated tablet weight (mg)** | **989** |

Thickness: 6.8 mm
Hardness: 30.6 N

### Example C4: Comparative solid pharmaceutical unit dosage formulation, namely tablet formulation containing 160 mg enzalutamide per tablet.

| **Ingredients** | **C4 (mg/unit)** |
|---|---|
| **Stage-A: (Dry Mix)** | |
| Enzalutamide, *In-house* | 161.208 |
| Cellulose Acetate Phthalate (CAP), *Ph. Eur.* | 640 |
| Triethyl citrate, *Ph. Eur.* | 175 |

| **Stage-B: (Hot Melt Extrusion)** | |
|---|---|
| **Total weight of Extrudes** | **976.208** |

| **Stage-C (Blending and Tablet Compression)** | |
|---|---|
| Silicified Microcrystalline Cellulose, *Ph. Eur.* | 148.792 |
| Low-substituted hydroxypropyl cellulose (L-HPC LH-21), *Ph. Eur.* | 95 |
| Magnesium stearate, *Ph. Eur.* | 10 |
| Crospovidone (Kollidon^{®} CL-M), *Ph. Eur.* | 120 |
| Lactose Monohydrate (FlowLac^{®} 90), *Ph. Eur.* | 200 |
| **Total weight of tablet (mg)** | **1550** |

Core Tablets
Thickness: 8.8 mm

### Part C: Process of production of inventive pharmaceutical tablet formulations containing enzalutamide by hot melt extrusion

The following inventive pharmaceutical tablet formulations **F1-F5** were produced in accordance with the general procedure as set out in Part A above. Different unit dosages of enzalutamide such as 40 mg, 80 mg, or 120 mg are produced by respective relative adapting the weight amounts of the constituents.

### Example F1: inventive solid pharmaceutical unit dosage formulation, namely tablet formulation containing 160 mg enzalutamide per tablet.

| **Ingredients** | **F1 (mg/unit)** |
|---|---|
| **Stage-A: (Dry Mix)** | |
| Enzalutamide, *In-house* | 161.208 |
| Hydroxypropyl Methylcellulose Phthalate (HPMCP HP-55), *Ph. Eur.* | 480 |
| Cellulose Acetate Phthalate, *Ph. Eur.* | 190 |
| Maize starch, *Ph. Eur.* | 43.23 |
| Triethyl citrate, *Ph. Eur.* | 43.23 |

| **Stage-B: (Hot Melt Extrusion)** | |
|---|---|
| **Total weight of Extrudes** | **917.67** |

| **Stage-C (Blending and Tablet Compression)** | |
|---|---|
| Silicified microcrystalline cellulose, *Ph. Eur.* | 148.33 |
| Low-substituted hydroxypropyl cellulose (L-HPC LH-21), *Ph. Eur.* | 100 |
| Magnesium stearate, *Ph. Eur.* | 10 |
| Crospovidone (Kollidon^{®} CL-M), *Ph. Eur.* | 104 |
| Lactose Monohydrate (FlowLac^{®} 90), *Ph. Eur.* | 100 |
| **Total weight of tablet (mg)** | **1,380.00** |

| **Stage-D (Film Coating)** | |
|---|---|
| Opadry^{®} white, *In-house* | **30.000** |
| Water, Purified, *Ph. Eur.* | **q.s.** |
| **Film-coated tablet weight (mg)** | **1,410.00** |

Tablets exhibited a thickness of 8 mm, tablet dimensions of 21.5 x 10.5 mm oval shape and a hardness between 120 and 180 N.

### Example F2: inventive solid pharmaceutical unit dosage formulation, namely film-coated tablet formulation containing 160 mg enzalutamide per tablet.

| **Ingredients** | **F2 (mg/unit)** |
|---|---|
| **Stage-A: (Dry Mix)** | |
| Enzalutamide, In-house | 161.21 |
| Hydroxypropyl Methylcellulose Phthalate (HPMCP HP-55), *Ph. Eur.* | 430 |
| Cellulose Acetate Phthalate (CAP), *Ph. Eur.* | 230 |
| Maize Starch, *Ph. Eur.* | 0 |
| Triethyl citrate, *Ph. Eur.* | 40.62 |
| **Stage-B: (Hot Melt Extrusion)** | |
| **Total weight of Extrudes** | **861.83** |

| **Stage-C (Blending and Tablet Compression)** | |
|---|---|
| Silicified Microcrystalline Cellulose, *Ph. Eur.* | 148.17 |
| Low-substituted hydroxypropyl cellulose (L-HPC LH-21), *Ph. Eur.* | 100 |
| Magnesium stearate, *Ph. Eur.* | 10 |
| Crospovidone (Kollidon^{®} CL-M), *Ph. Eur.* | 100 |
| Colloidal anhydrous silica (Aerosil^{®} 200), *Ph. Eur.* | 20 |
| Lactose monohydrate (FlowLac^{®} 90), *Ph. Eur.* | 100 |
| **Total weight of core tablet (mg)** | **1,340.00** |

| **Stage-D (Film Coating)** | |
|---|---|
| Opadry^{®} White, *In-house* | 30.000 |
| Water, Purified, *Ph. Eur.* | q.s. |
| **Film-coated tablet weight (mg)** | **1,370.00** |

Tablets exhibited a thickness of 8 mm, tablet dimensions of 21.5 x 10.5 mm oval shape and a hardness between 120 and 180 N.

### Example F3: inventive solid pharmaceutical unit dosage formulation, namely tablet formulation containing 160 mg enzalutamide per tablet.

| **Ingredients** | **F3 (mg/unit)** |
|---|---|
| **Stage-A: (Dry Mix)** | |
| Enzalutamide, *In-house* | 161.21 |
| Hydroxypropyl Methylcellulose Phthalate (HPMCP HP-55), *Ph. Eur.* | 480 |
| Cellulose Acetate Phthalate (CAP), *Ph. Eur.* | 320 |
| Anhydrous lactose, *Ph. Eur.* | 100 |
| Triethyl citrate, *Ph. Eur.* | 65 |

| **Stage-B: (Hot Melt Extrusion)** | |
|---|---|
| **Total weight of Extrudes** | **1,126.20** |

| **Stage-C (Blending and Tablet Compression)** | |
|---|---|
| Silicified microcrystalline cellulose, *Ph. Eur.* | 148.79 |
| Low-substituted hydroxypropyl cellulose (L-HPC LH-21), *Ph. Eur.* | 100 |
| Magnesium stearate, *Ph. Eur.* | 10 |
| Crospovidone (Kollidon^{®} CL-M), *Ph. Eur.* | 115 |
| Lactose Monohydrate (FlowLac^{®} 90), *Ph. Eur.* | 50 |
| **Total weight of core tablet (mg)** | **1,550.00** |

Core tablets exhibited a thickness of 8 mm tablet dimensions of 21.3 x 10.4 mm oval shape and a hardness between 120 and 180 N.

### Example F4: inventive solid pharmaceutical unit dosage formulation, namely film-coated tablet formulation containing 160 mg enzalutamide per tablet.

| **Ingredients** | **F4 (mg/unit)** |
|---|---|
| **Stage-A: (Dry Mix)** | |
| Enzalutamide, *In-house* | 161.21 |
| Hydroxypropyl Methylcellulose Phthalate (HPMCP HP-55), *Ph. Eur.* | 430 |
| Cellulose Acetate Phthalate (CAP), *Ph. Eur.* | 230 |
| Maize Starch, *Ph. Eur.* | 42.7 |
| Triethyl citrate, *Ph. Eur.* | 42.7 |

| **Stage-B: (Hot Melt Extrusion)** | |
|---|---|
| **Total weight of Extrudes** | **906.61** |

| **Stage-C (Blending and Tablet Compression)** | |
|---|---|
| Silicified Microcrystalline Cellulose, *Ph. Eur.* | 147.39 |
| Low-substituted hydroxypropyl cellulose (L-HPC LH-11), *Ph. Eur.* | 98 |
| Magnesium stearate, *Ph. Eur.* | 10 |
| Crospovidone (Kollidon^{®} CL-M), *Ph. Eur.* | 100 |
| Colloidal anhydrous silica (Aerosil^{®} 200), *Ph. Eur.* | 20 |
| Lactose Monohydrate (FlowLac^{®} 90), *Ph. Eur.* | 100 |
| **Total weight of core tablet (mg)** | **1,380.00** |

| **Stage-D (Film Coating)** | |
|---|---|
| Opadry^{®} White, *In-house* | 30.000 |
| Water, Purified, *Ph. Eur.* | q.s. |
| **Film-coated tablet weight (mg)** | **1,410.00** |

### Example F5: inventive solid pharmaceutical unit dosage formulation, namely film-coated tablet formulation containing 160 mg enzalutamide per tablet.

| **Ingredients** | **F5 (mg/unit)** |
|---|---|
| **Stage-A: (Dry Mix)** | |
| Enzalutamide, *In-house* | 160 |
| Hydroxypropyl Methylcellulose Phthalate (HPMCP HP-55), *Ph. Eur.* | 430 |
| Cellulose Acetate Phthalate, *Ph. Eur.* | 230 |
| Maize Starch, *Ph. Eur.* | 42.7 |
| Triethyl citrate, *Ph. Eur.* | 42.7 |
| Propyl Gallate, *Ph. Eur.* | 2 |

| Stage-B: (Hot Melt Extrusion) | |
|---|---|
| Total weight of Extrudes | 907.4 |

| Stage-C (Blending and Tablet Compression) | |
|---|---|
| Silicified microcrystalline cellulose, *Ph. Eur.* | 148.6 |
| Low-substituted hydroxypropyl cellulose (L-HPC LH-21), *Ph. Eur.* | 97 |
| Magnesium stearate, *Ph. Eur.* | 10 |
| Crospovidone (Kollidon^{®} CL-M) | 100 |
| Colloidal anhydrous silica (Aerosil^{®} 200), *Ph. Eur.* | 20 |
| Lactose Monohydrate (FlowLac^{®} 90) | 97 |
| **Total weight of core tablet (mg)** | **1,380.00** |

| **Stage-D (Film Coating)** | |
|---|---|
| Opadry^{®} White, *In-house* | 30.000 |
| Water, Purified, *Ph. Eur.* | q.s. |
| **Film-coated tablet weight (mg)** | **1,410.00** |

Tablets exhibited a thickness of 8 mm, tablet dimensions of 21.5 x 10.5 mm oval shape and a hardness between 120 and 180 N.

### Example F6: inventive solid pharmaceutical unit dosage formulation, namely film-coated tablet formulation containing 160 mg enzalutamide per tablet.

| **Ingredients** | **F6 (mg/unit)** |
|---|---|
| **Stage-A: (Dry Mix)** | |
| Enzalutamide, *In-house* | 160.19 |
| Hydroxypropyl Methylcellulose Phthalate (HPMCP HP-55S), *Ph. Eur.* | 640 |
| Cellulose Acetate Phthalate, *Ph. Eur.* | 150 |
| Maize Starch, *Ph. Eur.* | 41.15 |
| Triethyl citrate, *Ph. Eur.* | 61.65 |

| **Stage-B: (Hot Melt Extrusion)** | |
|---|---|
| **Total weight of Extrudes** | **1052.99** |

| **Stage-C (Blending and Tablet Compression)** | |
|---|---|
| Silicified microcrystalline cellulose, *Ph. Eur.* | 149.51 |
| Low-substituted hydroxypropyl cellulose (L-HPC LH-21), *Ph. Eur.* | 57.5 |
| Magnesium stearate, *Ph. Eur.* | 10 |
| Crospovidone (Kollidon^{®} CL-M) | 100 |
| Lactose Monohydrate (FlowLac^{®} 90) | 100 |
| **Total weight of core tablet (mg)** | **1,470.00** |

| **Stage-D (Film Coating)** | |
|---|---|
| Opadry^{®} White, *In-house* | 30.000 |
| Water, Purified, *Ph. Eur.* | q.s. |
| **Film-coated tablet weight (mg)** | **1,500.00** |

Tablets exhibited a thickness of 8.5 mm, tablet dimensions of 21.3 x 10.4 mm oval shape and a hardness between 155 and 188 N.

### Example F7: inventive solid pharmaceutical unit dosage formulation, namely film-coated tablet formulation containing 160 mg enzalutamide per tablet.

| **Ingredients** | **F7 (mg/unit)** |
|---|---|
| **Stage-A: (Dry Mix)** | |
| Enzalutamide, *In-house* | 160.19 |
| Hydroxypropyl Methylcellulose Phthalate (HPMCP HP-55S), *Ph. Eur.* | 640 |
| Cellulose Acetate Phthalate, *Ph. Eur.* | 175 |
| Maize Starch, *Ph. Eur.* | 41.15 |
| Triethyl citrate, *Ph. Eur.* | 50.65 |

| **Stage-B: (Hot Melt Extrusion)** | |
|---|---|
| **Total weight of Extrudes** | **1066.99** |

| **Stage-C (Blending and Tablet Compression)** | |
|---|---|
| Silicified microcrystalline cellulose, *Ph. Eur.* | 149.51 |
| Low-substituted hydroxypropyl cellulose (L-HPC LH-21), *Ph. Eur.* | 48.5 |
| Magnesium stearate, *Ph. Eur.* | 10 |
| Crospovidone (Kollidon^{®} CL-M) | 95 |
| Lactose Monohydrate (FlowLac^{®} 90) | 100 |
| **Total weight of core tablet (mg)** | **1,470.00** |

| **Stage-D (Film Coating)** | |
|---|---|
| Opadry^{®} White, *In-house* | 30.000 |
| Water, Purified, *Ph. Eur.* | q.s. |
| **Film-coated tablet weight (mg)** | **1,500.00** |

Tablets exhibited a thickness of 8.5 mm, tablet dimensions of 21.3 x 10.4 mm oval shape and a hardness between 160 and 180 N.

### Part D: General Procedure for the production of the amorphous solid dispersion by spray granulation I wet granulation and optional subsequent further processing

### Stage A: (Spray granulation)

1. Dissolving enzalutamide in a solvent, such as in acetone, and mixing the resulting solution with polymeric constituents hydroxypropyl methylcellulose phthalate and cellulose acetate phthalate, as well as optionally further pharmaceutical excipients, e.g. antioxidants, followed by spray granulation on a pharmaceutically acceptable carrier or wet granulation with a pharmaceutically acceptable carrier, such as diluents, disintegrants, and silica-based adsorbents, e.g., mixture of microcrystalline cellulose and crospovidone as a pharmaceutically acceptable carrier, preferably in a Fluid bed granulator, for a suitable time to produce an amorphous solid dispersion containing enzalutamide on the carrier.
2. The granules of step-1 were dried in a suitable equipment.
3. The dried granules of step-2 were milled, e.g., using cone mill equipped with suitable screen to form a solid pharmaceutical formulation selected from the group consisting of powder, granules, pellets, and beads.

### Optional Stage B: (Blending and further processing)

4. Providing one or more extragranular pharmaceutically acceptable constituents, such as selected from diluent agent, flow enhancer agent, disintegrant agent, binder agent, solubilizer agent, and lubricant agent, e.g. silicified microcrystalline cellulose, starch, low-substituted hydroxypropyl cellulose, lactose anhydrate, lactose monohydrate, and magnesium stearate.

5. Blending the granules of step-3 with the pharmaceutical excipients of step-4 in a mixer for a suitable amount of time, e.g., 10 minutes, and further processing the mixture to form a solid pharmaceutical unit dosage formulation, such as selected from the group consisting of tablets, film-coated tablets, capsules, caplets, multiple unit pellet systems.

6. As an example, the blend of step-5 is compressed into cores of tablets, e.g. on a rotary tablet compression machine using suitable tooling and parameters.

### Optional Stage C: (Film-Coating)

7. Film coating the core tablets of step-6, e.g. using a suitable coating pan, with a suitable film coating constituent, e.g. a suitable Opadry^{®} suspension.

### Part E: Process of production of inventive pharmaceutical tablet formulations containing enzalutamide by spray granulation

The following inventive pharmaceutical tablet formulation F6 was produced in accordance with the general procedure as set out in Part D above. Different unit dosages of enzalutamide such as 40 mg, 80 mg, or 120 mg are produced by respective relative adapting the weight amounts of the constituents.

### Example F8: inventive solid pharmaceutical unit dosage formulation, namely tablet formulation containing 160 mg enzalutamide per tablet.

| **Ingredients** | **F8 (mg/unit)** |
|---|---|
| **Stage-A: (Spray granulation)** | |
| Enzalutamide, *In-house* | 160 |
| Hydroxypropyl methylcellulose phthalate (HPMCP HP-55), *Ph. Eur.* | 420 |
| Cellulose Acetate Phthalate (CAP), *Ph. Eur.* | 130 |
| Microcrystalline cellulose (MCC 102), *Ph. Eur.* | 600 |
| Crospovidone (Kollidon^{®} CL-M), *Ph. Eur.* | 30 |
| Acetone | *q.s.* |
| **Total weight of granulated blend** | **1,340** |

| **Stage-B (Blending and tablet compressing)** | |
|---|---|
| Silicified microcrystalline cellulose, *Ph. Eur.* | 25 |
| Low-substituted hydroxypropyl cellulose (L-HPC LH-21), *Ph. Eur.* | 20 |
| Lactose Monohydrate (FlowLac^{®} 90), *Ph. Eur.* | 30 |
| Crospovidone (Kollidon^{®} CL-M), *Ph. Eur.* | 25 |
| Magnesium stearate, *Ph. Eur.* | 10 |
| **Total weight of tablet (mg)** | **1,450.00** |

### Part F: Prior Art Formulations

Prior art tablet formulations of enzalutamide are generally prepared by spray drying and contain 40 mg or 80 mg enzalutamide per film-coated tablet (commercialized under Xtandi^{®} 40 mg tablets in EU, Japan, and US, or Xtandi^{®} 80 mg tablets in Japan and US).

### Ingredients of Xtandi^{®} tablets containing 40 mg or 80 mg enzalutamide:

| **Ingredients Xtandi^{®}** |
|---|
| **Tablet Core** |
| Enzalutamide |
| Hypromellose acetate succinate |
| Microcrystalline Cellulose |
| Colloidal anhydrous silica |
| Croscarmellose sodium |
| Magnesium stearate |

| **Film coating** |
|---|
| Hypromellose |
| Talc |
| Macrogol (8000) |
| Titanium dioxide |
| Iron oxide yellow |

### Part G: Dissolution testing of inventive enzalutamide tablet compositions and comparative enzalutamide tablet compositions

For the tablet formulations of inventive and comparative examples, the dissolution test was carried out according to the following dissolution test methods and conditions.

Dissolution Method 1 (recommended by FDA Dissolution Method Database for Enzalutamide 40 mg tablets):
USP Apparatus II, paddle (50 RPM), 900 mL of phosphate buffer (pH 7.5). Specification: not less than 75% in 45 min.

Dissolution Method 2 (optimized *in-house* method suitable for 40-160 mg enzalutamide tablets):
USP Apparatus II, paddle (75 RPM), 900 mL of phosphate buffer (pH 7.5). Specification: not less than 75% in 45 min.

Dissolution Method 3 (QC release media):
USP Apparatus II, paddle (75 RPM), 900 mL of phosphate buffer pH 6.8 + 0.4% SLS. Specification: not less than 75% in 30 min.

**Table 1: Comparative dissolution profiles of Enzalutamide 160 mg tablets with Xtandi^{®} 40 mg using Dissolution Method 1**

| **Product/ Batch No.** | **Mean Cumulative % Labelled Amount Dissolved** | | | | | | |
|---|---|---|---|---|---|---|---|
| | **5 min** | **10 min** | **15 min** | **20 min** | **30 min** | **45 min** | **60 min** |
| **Xtandi^{®} 40 mg (Lot: 20F0401)** | 88 | 98 | 102 | 102 | 102 | **104** | 103 |
| **F3** | 39 | 54 | 62 | 66 | 71 | **79** | 83 |
| **C1** | 8 | 14 | 19 | 22 | 24 | **25** | 25 |
| **C2** | 23 | 22 | 26 | 25 | 24 | **13** | 8 |
| **C3** | 35 | 40 | 41 | 43 | 41 | **42** | 44 |
| **C4** | 31 | 40 | 44 | 46 | 52 | **57** | 58 |

The experimental data of Table 1 above shows that 75 wt.% or more of enzalutamide of the inventive pharmaceutical composition in tablet unit dosage formulation **F3** is dissolved within 45 minutes in accordance with the regulatory specification.

The dissolution rate for the respective comparative pharmaceutical tablet unit dosage formulation examples **C1**, **C2**, **C3**, and **C4** is slower, and the required 75 wt.% drug release is not achieved at 45 minute time point.

Furthermore, with respect to the comparative pharmaceutical tablet unit dosage formulation example **C2** containing HPMCP as sole polymer, the inventors have observed as a further drawback a strong reprecipitation (i.e., the drug release is falling from the maximal value of 26 wt.% at 15 minutes to only 8 wt.% at 60 minutes).

**Table 2: Comparative dissolution profiles of Enzalutamide 160 mg tablets with Xtandi^{®} 40 mg using Dissolution Method 2**

| **Product/ Batch No.** | **Mean Cumulative % Labelled Amount Dissolved** | | | | | | |
|---|---|---|---|---|---|---|---|
| | **5 min** | **10 min** | **15 min** | **20 min** | **30 min** | **45 min** | **60 min** |
| **Xtandi^{®} 40 mg (Lot: 22D0619)** | 79 | 91 | 93 | 96 | 98 | **100** | 100 |
| **F1** | 37 | 87 | 95 | 95 | 96 | **93** | 84 |
| **F2** | 79 | 86 | 90 | 88 | 87 | **85** | 84 |
| **F4** | 63 | 85 | 90 | 93 | 91 | **92** | 89 |
| **F5** | 81 | 94 | 93 | 92 | 89 | **88** | 88 |
| **F6** | 68 | 89 | 96 | 96 | 96 | **96** | 92 |
| **F7** | 69 | 82 | 89 | 93 | 93 | **93** | 92 |

The experimental data of Table 2 above shows that 75 wt.% or more of enzalutamide of the inventive pharmaceutical composition in tablet unit dosage formulations **F1**, **F2**, **F4**, **F5**, **F6**, and **F7** is dissolved within 45 minutes in accordance with the regulatory specification. Furthermore, the dissolution rate of the respective tablet unit dosage formulations **F1**, **F2**, **F4**, **F5**, **F6**, and **F7** of the present invention is comparable to the commercially available reference product (Xtandi^{®} 40 mg film-coated tablets). In particular, as more than 85 wt.% of enzalutamide is released already within 15 minutes with respect to the inventive pharmaceutical composition, all dissolution profiles of the inventive solid pharmaceutical tablet formulations and the reference product are respectively considered similar without the need to apply the similarity factor f₂ for calculating the dissolution similarity.

**Table 3: Comparative dissolution profiles of Enzalutamide 160 mg tablets with Xtandi^{®} 40 mg in Dissolution Method 3**

| **Product/ Batch No.** | **Mean Cumulative % Labelled Amount Dissolved** | | | | | | |
|---|---|---|---|---|---|---|---|
| | **5 min** | **10 min** | **15 min** | **20 min** | **30 min** | **45 min** | **60 min** |
| **Xtandi^{®} 40 mg (Lot# 22D0619)** | 95 | 100 | 100 | 100 | **100** | 100 | 100 |
| **F5** | 89 | 98 | 100 | 101 | **101** | 102 | 103 |

The experimental data of Table 3 above shows that 75 wt.% or more of enzalutamide of the inventive pharmaceutical composition in tablet unit dosage formulation **F5** is dissolved within 45 minutes in accordance with the regulatory specification. Furthermore, also the dissolution of the inventive tablet unit dosage formulation **F5** is comparable to the commercially available reference product (Xtandi^{®} 40 mg film-coated tablets). In particular, the dissolution profile of **F5** with the reference product Xtandi^{®} can be considered similar, as more than 85% of enzalutamide is released within 15 minutes and, thus, it is not required to apply the similarity factor f₂ for calculating the dissolution similarity.

### Part H: Stability testing of inventive enzalutamide tablet compositions

Stability and dissolution testings were performed on the inventive Enzalutamide 160 mg film-coated tablets. Inventive tablets **F1** were packed in PVC/PCTFE-Alu blister and stored at 30°C ± 2°C / 75% ± 5% relative humidity (RH) (ICH intermediate conditions) as well as at 40°C ± 2°C / 75% ± 5% RH (ICH accelerated conditions)

**Table 4: ICH Stability Data for storage at 30°C / 75% RH and 40°C / 75% RH**

| **Test** | **Tentative specification** | | **Initial** | **1 month 30°C / 75% RH** | **1 month 40°C / 75% RH** |
|---|---|---|---|---|---|
| **Description** | White to off white, oval shaped, film-coated tablets, plain on both sides. | | Complies | Complies | Complies |
| **Identification HPLC** | The retention time of enzalutamide peak of the sample solution should correspond to that of the standard solution as obtained in assay | | Complies | Complies | Complies |
| **Dissolution (% Labelled amount, by HPLC)** | Not less than 75 % of the labelled amount of enzalutamide is dissolved in 30 minutes (Dissolution Method 3) | Mean | 96 | 97 | 99 |

| **Related substances (%, by HPLC)** | | | | | |
|---|---|---|---|---|---|
| **a) Oxo impurity** | Not more than 0.2 | | BDL | BDL | 0.06 |
| **b) Des fluoro impurity** | Not more than 0.2 | | 0.06 | 0.06 | 0.06 |
| **c) Impurity-B** | Not more than 0.2 | | BDL | BDL | BDL |
| **d) Single maximum unspecified impurity** | Not more than 0.2 | | BDL | BDL | BDL |
| **e) total impurities** | Not more than 0.2 | | 0.06 | 0.06 | 0.12 |
| **Assay (% Label claim, by HPLC)** | 95-105 | | 99.3 | 98.6 | 99.5 |

As shown in Table 4 above, the inventive pharmaceutical composition in tablet unit dosage formulation **F1** fulfils the requirements of stability and dissolution after storage conditions at 30°C / 75% RH (intermediate ICH storage condition) and at 40°C / 75% RH (accelerated ICH storage condition) after 1 month.

Further embodiments of the present invention are listed below:
1. A pharmaceutical composition comprising or consisting of an amorphous solid dispersion of enzalutamide as active agent in a mixture of hydroxypropyl methylcellulose phthalate and cellulose acetate phthalate as pharmaceutically acceptable excipients.
2. The pharmaceutical composition according to embodiment 1, wherein the amorphous solid dispersion is prepared by hot melt extrusion.
3. The pharmaceutical composition according to embodiment 2, wherein the amorphous solid dispersion further comprises starch as a pharmaceutically acceptable excipient.
4. The pharmaceutical composition according to embodiment 3, wherein the starch is selected from one or more pharmaceutically acceptable excipients including the group consisting of maize starch, wheat starch, and potato starch.
5. The pharmaceutical composition according to any one of embodiments 2 to 4, wherein the amorphous solid dispersion further comprises a plasticizer as a pharmaceutically acceptable excipient.
6. The pharmaceutical composition according to embodiment 5, wherein the plasticizer is selected from one or more pharmaceutically acceptable excipients including the group consisting of triethyl citrate, triacetin, dibutyl sebacate, diethyl phthalate, glyceryl monostearate, glycerin, polyethylene glycol, and propylene glycol.
7. The pharmaceutical composition according to any one of the preceding embodiments, wherein the amorphous solid dispersion further comprises an antioxidant.
8. The pharmaceutical composition according to embodiment 7, wherein the antioxidant is propyl gallate.
9. The pharmaceutical composition according to any one of the preceding embodiments, wherein the amorphous solid dispersion contains:
   a. an amount of enzalutamide in the range of 10 to 25 wt.%, preferably 15 to 20 wt.% respectively based on the total weight of the amorphous solid dispersion, and/or
   b. an amount of hydroxypropyl methylcellulose phthalate in the range of 30 to 70 wt.%, preferably 40 to 60 wt.%, more preferably 45 to 55 wt.% respectively based on the total weight of the amorphous solid dispersion, and/or
   c. an amount of cellulose acetate phthalate in the range of 10 to 50 wt.%, preferably 20 to 35 wt.%, more preferably 25 to 30 wt.% respectively based on the total weight of the amorphous solid dispersion, and/or
   d. a ratio between hydroxypropyl methylcellulose phthalate and cellulose acetate phthalate in the range of 6 : 1 to 0.5 : 1, preferably 1.5 : 1 to 2.5 : 1 respectively based on the total weight of the amorphous solid dispersion, and/or
   e. an amount of starch in the range of 0 to 10 wt.%, preferably 3 to 8 wt.%, more preferably 4 to 6 wt.% respectively based on the total weight of the amorphous solid dispersion, and/or
   f. an amount of plasticizer in the range of 0 to 10 wt.%, preferably 3 to 8 wt.%, more preferably 4 to 6 wt.% respectively based on the total weight of the amorphous solid dispersion, and/or
   g. a ratio between starch to plasticizer in the range of 8 : 0 to 0 : 10, preferably 0.5 : 1 to 1.5 : 1, more preferably 1 : 1 respectively based on the total weight of the amorphous solid dispersion, and/or
   h. an amount of propyl gallate in a range of 0 to 1 wt.%, preferably 0.1 to 0.8 wt.%, more preferably 0.2 to 0.7 wt.% respectively based on the total weight of the amorphous solid dispersion.
10. The pharmaceutical composition according to embodiment 1, wherein the amorphous solid dispersion is prepared by spray drying, drum drying or co-precipitation.
11. The pharmaceutical composition according to embodiment 1, wherein the amorphous solid dispersion is prepared by spray granulation on a pharmaceutically acceptable carrier or wet granulation with a pharmaceutically acceptable carrier.
12. The pharmaceutical composition according to embodiment 11, wherein the pharmaceutically acceptable carrier is selected from one or more pharmaceutically acceptable excipients selected from the group of diluents, disintegrants, and silica-based adsorbents.
13. The pharmaceutical composition according to embodiment 12, wherein the silica-based adsorbent is selected from one or more pharmaceutically acceptable excipients of the group consisting of a mesoporous silica, colloidal anhydrous silica, magnesium aluminosilicate, and calcium silicate.
14. The pharmaceutical composition according to any one of the preceding embodiments, wherein the amorphous solid dispersion is further processed to form a solid pharmaceutical formulation selected from the group consisting of powder, granules, pellets, and beads.
15. The pharmaceutical composition according to embodiment 12 or 13, wherein the solid pharmaceutical formulation is further processed with one or more further pharmaceutically acceptable excipients selected from the group consisting of diluent agent, flow enhancer agent, disintegrant agent, binder agent, solubilizer agent, and lubricant agent to form a solid pharmaceutical unit dosage formulation selected from the group consisting of tablets, film-coated tablets, capsules, caplets, multiple unit pellet systems.
16. The pharmaceutical composition according to embodiment 15, wherein the unit dosage form contains 40 mg, 80 mg, 120 mg or 160 mg enzalutamide as active ingredient per unit dosage form.
17. The pharmaceutical composition according to any one of embodiments 1 to 14, wherein the solid pharmaceutical formulation or the solid pharmaceutical unit dosage form comprises one or more further pharmaceutically acceptable excipients selected from the group consisting of (silicified) microcrystalline cellulose, hydroxypropyl cellulose, low-substituted hydroxypropyl cellulose, methylcellulose, hydroxypropyl methylcellulose, hydroxypropyl methylcellulose acetate succinate, croscarmellose sodium, maltodextrin, starch, sodium starch glycolate, pregelatinized starch, crospovidone, lactose monohydrate, mannitol, magnesium stearate, colloidal anhydrous silica, sodium stearyl fumarate, and sodium carbonate, more preferably (silicified) microcrystalline cellulose, low-substituted hydroxypropyl cellulose, lactose monohydrate, magnesium stearate, colloidal anhydrous silica, croscarmellose sodium, and crospovidone.
18. A solid pharmaceutical composition according to any one of the preceding embodiments for use in the treatment of a hyperproliferative disorder, preferably for use in the treatment of
   - adult men with non-metastatic castration-resistant prostate cancer (CRPC),
   - adult men with metastatic CRPC who are asymptomatic or mildly symptomatic after failure of androgen deprivation therapy in whom chemotherapy is not yet clinically indicated, or
   - adult men with metastatic CRPC whose disease has progressed on or after docetaxel therapy.
19. Process of production of a solid pharmaceutical composition prepared according to hot melt extrusion containing enzalutamide as active ingredient and pharmaceutically acceptable excipients in an amorphous solid dispersion, characterized in that the process comprises or consists of the following steps
   a. mixing a pharmaceutical amount of enzalutamide as active ingredient together with pharmaceutically acceptable excipients comprising or consisting of hydroxypropyl methylcellulose phthalate and cellulose acetate phthalate,
   b. heating the mixture of step a) to form a melt and extruding the formed melt followed by subsequent cooling the extrudate to form an amorphous solid dispersion comprising or consisting of enzalutamide, hydroxypropyl methylcellulose phthalate and cellulose acetate phthalate,
   c. optionally further processing the amorphous solid dispersion of step b) to form a solid pharmaceutical formulation selected from the group consisting of a powder, granules, pellets, and beads, and
   d. optionally further processing the solid pharmaceutical formulation of step c) with one or more further pharmaceutically acceptable excipients selected from the group consisting of diluent agent, flow enhancer agent, disintegrant agent, binder agent, solubilizer agent, and lubricant agent to form a solid pharmaceutical unit dosage formulation selected from the group consisting of tablets, film-coated tablets, capsules, caplets, and multiple unit pellet systems.
20. A method of treatment or prophylaxis of a hyperproliferative disorder in a patient suffering from, or at risk of, said disorder or condition, which comprises administering to the patient a pharmaceutical composition according to any one of embodiments 1 to 17 or according to embodiment 18 or prepared according to embodiment 19, wherein the patient is selected from an
   - adult men with non-metastatic castration-resistant prostate cancer (CRPC),
   - adult men with metastatic CRPC who are asymptomatic or mildly symptomatic after failure of androgen deprivation therapy in whom chemotherapy is not yet clinically indicated, or
   - adult men with metastatic CRPC whose disease has progressed on or after docetaxel therapy.

## Claims

1. A pharmaceutical composition comprising or consisting of an amorphous solid dispersion of enzalutamide as active agent in a mixture of hydroxypropyl methylcellulose phthalate and cellulose acetate phthalate as pharmaceutically acceptable excipients.

2. The pharmaceutical composition according to claim 1, wherein a ratio between the enzalutamide as active agent and the mixture of hydroxypropyl methylcellulose phthalate and cellulose acetate phthalate in the amorphous solid dispersion is in the range of 1 : 4 to 1 : 6, preferably 1 : 5 respectively based on the total weight of the amorphous solid dispersion and wherein the amorphous solid dispersion is prepared by hot melt extrusion.

3. The pharmaceutical composition according to claim 2, wherein the amorphous solid dispersion further comprises starch as a pharmaceutically acceptable excipient.

4. The pharmaceutical composition according to claim 3, wherein the starch is selected from one or more pharmaceutically acceptable excipients including the group consisting of maize starch, wheat starch, and potato starch.

5. The pharmaceutical composition according to any one of claims 2 to 4, wherein the amorphous solid dispersion further comprises a plasticizer as a pharmaceutically acceptable excipient.

6. The pharmaceutical composition according to claim 5, wherein the plasticizer is selected from one or more pharmaceutically acceptable excipients including the group consisting of triethyl citrate, triacetin, dibutyl sebacate, diethyl phthalate, glyceryl monostearate, glycerin, polyethylene glycol, and propylene glycol.

7. The pharmaceutical composition according to any one of the preceding claims, wherein the amorphous solid dispersion contains:
a. an amount of enzalutamide in the range of 10 to 25 wt.%, preferably 15 to 20 wt.% respectively based on the total weight of the amorphous solid dispersion, and/or
b. an amount of hydroxypropyl methylcellulose phthalate in the range of 30 to 70 wt.%, preferably 55 to 70 wt.%, more preferably 56 to 64 wt.% or 59 to 62 wt.% respectively based on the total weight of the amorphous solid dispersion, and/or
c. an amount of cellulose acetate phthalate in the range of 10 to 50 wt.%, preferably 10 to 20 wt.%, more preferably 11 to 19 wt.% or 14 to 16 wt.% respectively based on the total weight of the amorphous solid dispersion, and/or
d. a ratio between hydroxypropyl methylcellulose phthalate and cellulose acetate phthalate in the range of 6 : 1 to 0.5 : 1, preferably 4.5 : 1 to 3.5 : 1 respectively based on the total weight of the amorphous solid dispersion, and/or
e. an amount of starch in the range of 0 to 10 wt.%, preferably 3 to 8 wt.%, more preferably 3 to 4 wt.% respectively based on the total weight of the amorphous solid dispersion, and/or
f. an amount of plasticizer in the range of 0 to 10 wt.%, preferably 3 to 8 wt.%, more preferably 4 to 6 wt.% respectively based on the total weight of the amorphous solid dispersion, and/or
g. a ratio between starch to plasticizer in the range of 8 : 0 to 0 : 10, preferably 0.5 : 1 to 1.5 : 1, more preferably 1 : 1.5 to 1 : 1 respectively based on the total weight of the amorphous solid dispersion, and/or
h. an amount of propyl gallate in a range of 0 to 1 wt.%, preferably 0.1 to 0.8 wt.%, more preferably 0.2 to 0.7 wt.% respectively based on the total weight of the amorphous solid dispersion.

8. The pharmaceutical composition according to claim 1, wherein the amorphous solid dispersion is prepared by spray drying, drum drying or co-precipitation.

9. The pharmaceutical composition according to claim 1, wherein the amorphous solid dispersion is prepared by spray granulation on a pharmaceutically acceptable carrier or wet granulation with a pharmaceutically acceptable carrier.

10. The pharmaceutical composition according to claim 9, wherein the pharmaceutically acceptable carrier is selected from one or more pharmaceutically acceptable excipients selected from the group of diluents, disintegrants, and silica-based adsorbents.

11. The pharmaceutical composition according to claim 10, wherein the silica-based adsorbent is selected from one or more pharmaceutically acceptable excipients of the group consisting of a mesoporous silica, colloidal anhydrous silica, magnesium aluminosilicate, and calcium silicate.

12. The pharmaceutical composition according to any one of the preceding claims, wherein the amorphous solid dispersion is further processed to form a solid pharmaceutical formulation selected from the group consisting of powder, granules, pellets, and beads.

13. The pharmaceutical composition according to claim 10 or 11, wherein the solid pharmaceutical formulation is further processed with one or more further pharmaceutically acceptable excipients selected from the group consisting of diluent agent, flow enhancer agent, disintegrant agent, binder agent, solubilizer agent, and lubricant agent to form a solid pharmaceutical unit dosage formulation selected from the group consisting of tablets, film-coated tablets, capsules, caplets, multiple unit pellet systems.

14. The pharmaceutical composition according to claim 13, wherein the unit dosage form contains 40 mg, 80 mg, 120 mg or 160 mg enzalutamide as active ingredient per unit dosage form.

15. The pharmaceutical composition according to any one of claims 1 to 12, wherein the solid pharmaceutical formulation or the solid pharmaceutical unit dosage form comprises one or more further pharmaceutically acceptable excipients selected from the group consisting of (silicified) microcrystalline cellulose, hydroxypropyl cellulose, low-substituted hydroxypropyl cellulose, methylcellulose, hydroxypropyl methylcellulose, hydroxypropyl methylcellulose acetate succinate, croscarmellose sodium, maltodextrin, starch, sodium starch glycolate, pregelatinized starch, crospovidone, lactose monohydrate, mannitol, magnesium stearate, colloidal anhydrous silica, sodium stearyl fumarate, and sodium carbonate, more preferably (silicified) microcrystalline cellulose, low-substituted hydroxypropyl cellulose, lactose monohydrate, magnesium stearate, colloidal anhydrous silica, croscarmellose sodium, and crospovidone.

16. The pharmaceutical composition according to claim 15, wherein the solid pharmaceutical formulation or the solid pharmaceutical unit dosage form comprises or consists of enzalutamide, hydroxypropyl methylcellulose phthalate, cellulose acetate phthalate, maize starch, triethyl citrate respectively forming the amorphous solid dispersion and silicified microcrystalline cellulose, low-substituted hydroxypropyl cellulose, magnesium stearate, crospovidone and lactose monohydrate.

17. The pharmaceutical composition according to claim 16, wherein the ratio between the enzalutamide as active agent and the mixture of hydroxypropyl methylcellulose phthalate and cellulose acetate phthalate in the amorphous solid dispersion is in the range of 1 : 4 to 1 : 6, preferably 1 : 5 respectively based on the total weight of the amorphous solid dispersion and wherein the ratio between hydroxypropyl methylcellulose phthalate and cellulose acetate phthalate in the amorphous solid dispersion is in the range of 4.5 : 1 to 3.5 : 1 respectively based on the total weight of the amorphous solid dispersion.

18. A solid pharmaceutical composition according to any one of the preceding claims for use in the treatment of a hyperproliferative disorder, preferably for use in the treatment of
- adult men with non-metastatic castration-resistant prostate cancer (CRPC),
- adult men with metastatic CRPC who are asymptomatic or mildly symptomatic after failure of androgen deprivation therapy in whom chemotherapy is not yet clinically indicated, or
- adult men with metastatic CRPC whose disease has progressed on or after docetaxel therapy.

19. Process of production of a solid pharmaceutical composition prepared according to hot melt extrusion containing enzalutamide as active ingredient and pharmaceutically acceptable excipients in an amorphous solid dispersion, **characterized in that** the process comprises or consists of the following steps
a. mixing a pharmaceutical amount of enzalutamide as active ingredient together with pharmaceutically acceptable excipients comprising or consisting of hydroxypropyl methylcellulose phthalate and cellulose acetate phthalate,
b. heating the mixture of step a) to form a melt and extruding the formed melt followed by subsequent cooling the extrudate to form an amorphous solid dispersion comprising or consisting of enzalutamide, hydroxypropyl methylcellulose phthalate and cellulose acetate phthalate,
c. optionally further processing the amorphous solid dispersion of step b) to form a solid pharmaceutical formulation selected from the group consisting of a powder, granules, pellets, and beads, and
d. optionally further processing the solid pharmaceutical formulation of step c) with one or more further pharmaceutically acceptable excipients selected from the group consisting of diluent agent, flow enhancer agent, disintegrant agent, binder agent, solubilizer agent, and lubricant agent to form a solid pharmaceutical unit dosage formulation selected from the group consisting of tablets, film-coated tablets, capsules, caplets, and multiple unit pellet systems.
